# EUROPÄISCHE PATENTSCHRIFT

(11) **EP 2 941 122 B1**
(45) Veröffentlichungstag und Bekanntmachung des Hinweises auf die Patenterteilung: **26.04.2017**
(21) Anmeldenummer: 14711732.9
(22) Anmeldetag: 17.03.2014
(51) Int. Cl.: A01K 29/00, A01K 43/00, A01K 45/00, A61B 5/055, A61B 5/145

(54) **VERFAHREN ZUM BESTIMMEN DES GESCHLECHTS EINES EMBRYOS IN EINEM EI**
METHOD OF DETERMINING THE GENDER OF AN EMBRYO IN AN EGG
PROCÉDÉ DE DÉTERMINATION DU SEXE D'UN EMBRYON ENCORE DANS L'OEUF

(30) Priorität: 27.03.2013 DE 102013205426
(43) Veröffentlichungstag der Anmeldung: 11.11.2015
(73) Patentinhaber: Siemens Healthcare GmbH, 91052 Erlangen (DE)
(72) Erfinder: SEWIOLO, Benjamin, 90587 Obermichelbach OT Rothenberg (DE); ZIROFF, Andreas, 80469 München (DE)
(86) Internationale Anmeldenummer: PCT/EP2014/055278
(87) Internationale Veröffentlichungsnummer: WO 2014/154513

(56) Entgegenhaltungen:
- WO-A1-98/14781
- US-B1- 7 950 349

## Beschreibung

Verfahren zum Bestimmen des Geschlechts eines Embryos in einem Vogelei. Die Erfindung betrifft ein Verfahren zum Bestimmen des Geschlechts eines Embryos in einem Vogelei, insbesondere einem Hühnerei, gemäß dem Oberbegriff von Patentanspruch 1.

In der Geflügelzucht sind männliche Küken ökonomisch unerwünscht, da sie bzw. Hähne keine Eier legen und kaum an Gewicht zunehmen. Daher werden männliche Küken nach dem Schlüpfen üblicherweise aussortiert und gekeult. Zusätzlich zu den Kosten zum Gewährleisten von Tierschutzaspekten fallen außerdem zusätzliche Kosten an, da alle Eier, seien es Eier mit Embryos männlichen Geschlechts, seien es Eier mit Embryos weiblichen Geschlechts, bebrütet werden müssen. Eine Geschlechtsbestimmung im Ei könnte dies verhindern.

In der Forschung existieren bereits sehr aufwändige Methoden, um das Geschlecht des Embryos im Ei, insbesondere im Hühnerei, mittels bezogen auf das Ei invasiven Verfahren zu bestimmen. Dabei wird beispielsweise mittels eines Lasers ein Loch mit einer Größe von wenigen Millimetern in die Eierschale eingebracht. Mit Hilfe von OCT (Optischer Kohärenztomographie) wird die Keimscheibe im Ei berührungsfrei lokalisiert. Schließlich wird mit Hilfe von Infrarotspektroskopie das Geschlecht bestimmt.

Die WO 98/14781 A1 offenbart ein Verfahren zum Bestimmen des Geschlechts wenigstens eines Embryos in einem Ei, bei welchem das Geschlecht des Embryos mittels wenigstens eines zumindest bezogen auf das Ei nichtinvasiven Ermittlungsverfahrens bestimmt wird. Mittels des Ermittlungsverfahrens wird ein Gehalt eines Östrogens in dem Ei bestimmt, wobei anhand des bestimmten Gehalts an Östrogen das Geschlecht des Embryos bestimmt wird. Bei dem Östrogen kann es sich um Östradiol handeln.

Der US 6,029,089 ist ein Verfahren zum Bestimmen des Geschlechts wenigstens eines Embryos in einem Ei, insbesondere in einem Hühnerei, als bekannt zu entnehmen. Bei dem Verfahren wird das Geschlecht des Embryos mittels wenigstens eines bezogen auf das Ei nichtinvasiven Ermittlungsverfahrens bestimmt. Als das nichtinvasive Ermittlungsverfahren wird beispielsweise ein Magnetresonanztomographie-Verfahren durchgeführt. Mittels des nichtinvasiven Ermittlungsverfahrens werden die Geschlechtsorgane des Embryos untersucht. Anhand dieser Untersuchung wird schließlich das Geschlecht des Embryos bestimmt.

Auch dieses Verfahren ist aufwändig und somit kostenintensiv, außerdem muss das Ei sehr lange bebrütet werden, um im Anschluss daran die Geschlechtsorgane des zumindest teilweise ausgebildeten Embryos zu untersuchen.

Es ist daher Aufgabe der vorliegenden Erfindung, ein Verfahren zur Geschlechtsbestimmung in einem Vogelei, insbesondere in einem Hühnerei, der eingangs genannten Art derart weiterzuentwickeln, dass eine besonders frühe Bestimmung des Geschlechts auf besonders einfache und somit zeit- und kostengünstige Weise möglich ist.

Diese Aufgabe wird durch ein Verfahren mit den Merkmalen des Patentanspruchs 1 gelöst. Vorteilhafte Ausgestaltungen mit zweckmäßigen und nicht-trivialen Weiterbildungen der Erfindung sind in den übrigen Ansprüchen angegeben.

Bei einem solchen Verfahren zum Bestimmen des Geschlechts wenigstens eines Embryos in einem Vogelei und insbesondere in einem Hühnerei, wird mittels wenigstens eines zumindest bezogen auf das Ei nichtinvasiven Ermittlungsverfahrens das Geschlecht des Embryos bestimmt.

Zur Realisierung einer besonders einfachen, zeit- und kostengünstigen sowie bezogen auf das Alter des Eis besonders frühzeitigen Geschlechtsbestimmung, ist es vorgesehen, dass mittels des nichtinvasiven Ermittlungsverfahrens wenigstens ein die Konzentration von Östradiol in dem Ei charakterisierender Östradiolwert ermittelt wird. In Abhängigkeit von dem ermittelten Östradiolwert wird das Geschlecht bestimmt.

Erfindungsgemäß wird mittels des nichtinvasiven Ermittlungsverfahrens, insbesondere mittels des bildgebenden Verfahrens und insbesondere mittels der Magnetresonanzspektroskopie die chemische Verschiebung von Wasserstoffatomen und/oder von Kohlenstoffatomen im Ei ermittelt, wobei in Abhängigkeit von der ermittelten Verschiebung der Östradiolwert ermittelt wird. Hierdurch können besonders präzise Rückschlüsse auf den Östradiolwert und schließlich auf das erwartete Geschlecht des sich zukünftig gegebenenfalls entwickelnden Embryos gezogen werden.

Unter der mittels des erfindungsgemäßen Verfahrens realisierbaren Bestimmung des Geschlechts des Embryos ist zu verstehen, dass der Embryo nicht bzw. noch nicht in dem Ei vorliegen muss. Mit anderen Worten muss der Embryo nicht oder nicht vollständig ausgebildet sein. Ist der Embryo nicht bzw. noch nicht oder nicht vollständig ausgebildet, so ist unter dem Bestimmen des Geschlechts des Embryos zu verstehen, dass das Geschlecht des sich gegebenenfalls zukünftig entwickelnden Embryos bestimmt wird. Das erfindungsgemäße Verfahren ermöglicht es nämlich, das Geschlecht zu bestimmen, ohne dass sich der Embryo bereits entwickelt hat. Mit anderen Worten kann das Verfahren in einem Zustand bzw. in einem Alter des Eis durchgeführt werden, in welchem sich der Embryo noch überhaupt nicht, lediglich teilweise oder bereits vollständig entwickelt hat. Vorzugsweise wird das Verfahren durchgeführt, wenn sich der Embryo noch nicht oder nur teilweise entwickelt hat. Dies bedeutet, dass das Geschlecht des sich zukünftig gegebenenfalls entwickelnden Embryos bzw. das Geschlecht bestimmt wird, welches der Embryo haben könnte, wenn er sich entwickeln würde.

Östradiol ist ein im Ei vorliegendes, weibliches Geschlechtshormon, dessen Konzentration im Ei mittels des nichtinvasiven Ermittlungsverfahrens ermittelt wird. Durch die Ermittlung der Konzentration des weiblichen Geschlechtshormons Östradiol kann das Geschlecht des sich insbesondere erst später, d.h. in Zukunft gegebenenfalls entwickelnden Embryos besonders frühzeitig und mit einer sehr hohen Wahrscheinlichkeit korrekt bestimmt werden. In der Folge kann der Aufwand der eingangs geschilderten Maßnahmen vermieden oder zumindest gering gehalten werden, da die entsprechenden Maßnahmen vor dem Schlüpfen des sich aus dem Ei entwickelnden Tieres, insbesondere des Kükens, ergriffen werden können.

Darüber hinaus ist es im Rahmen des erfindungsgemäßen Verfahrens nicht vorgesehen und nicht erforderlich, die Schale des Eis zu öffnen und Komponenten oder Substanzen aus dem Ei zu entnehmen. Dadurch kann der Zeit- und Kostenaufwand zur Geschlechtsbestimmung besonders gering gehalten werden.

In besonders vorteilhafter Ausgestaltung der Erfindung wird als das nichtinvasive Ermittlungsverfahren ein bildgebendes Verfahren, insbesondere ein Magnetresonanzspektroskopie-Verfahren, durchgeführt. Hierdurch kann die Konzentration von Östradiol im Ei besonders schnell und präzise ermittelt werden, so dass das Geschlecht bzw. das erwartete Geschlecht mit einer sehr hohen Wahrscheinlichkeit korrekt bestimmt werden kann.

Als zweckmäßig hat es sich gezeigt, die chemische Verschiebung in der Einheit Parts per Million (ppm) zu ermitteln. Zur Ermittlung der chemischen Verschiebung von Wasserstoffatomen im Ei wird beispielsweise eine ¹H-Spektroskopie durchgeführt. Zur Ermittlung der chemischen Verschiebung von Kohlenstoffatomen in dem Ei wird beispielsweise eine ¹³C-Spektroskopie durchgeführt. Dadurch kann der Östradiolwert besonders genau ermittelt werden, so dass wiederum das Geschlecht mit einer sehr hohen Wahrscheinlichkeit korrekt bestimmt werden kann. Bei einer besonders vorteilhaften Ausführungsform der Erfindung wird mittels des nichtinvasiven Ermittlungsverfahrens wenigstens ein die Konzentration von Testosteron in dem Ei charakterisierender Testosteronwert ermittelt. Ferner wird ein Verhältnis des Östradiolwerts zu dem ermittelten Testosteronwert ermittelt, wobei das Geschlecht in Abhängigkeit von dem Vergleich bestimmt wird. Mit anderen Worten wird das weibliche Geschlechtshormon Östradiol mit dem männlichen Geschlechtshormon Testosteron in dem Ei verglichen. Anhand dieses Vergleichs kann das Geschlecht dann mit einer sehr hohen Wahrscheinlichkeit korrekt bestimmt werden, bevor sich der Embryo und insbesondere das Küken teilweise oder vollständig entwickelt haben.

Zur Realisierung besonders aussagekräftiger Ergebnisse hat es sich als besonders vorteilhaft gezeigt, wenn der Östradiolwert und gegebenenfalls der Testosteronwert vor dem dreißigsten Bruttag des Eis ermittelt werden.

Das Verfahren ermöglicht auch besonders gute Ergebnisse, wenn der Östradiolwert und gegebenenfalls der Testosteronwert vor dem fünfundzwanzigsten, insbesondere ungefähr am siebzehnten, Bruttag ermittelt werden. Hierdurch können die gegebenenfalls und je nach ermitteltem Geschlecht durchzuführenden und auf das Ei bezogenen Maßnahmen besonders frühzeitig durchgeführt werden, so dass sich die Kosten gering halten und ein sehr guter Tierschutzaspekt realisieren lassen.

Als besonders zweckmäßig hat es sich gezeigt, den Östradiolwert und den Testosteronwert am gleichen Bruttag des Eis zu ermitteln. Hierdurch können der Östradiolwert und der Testosteronwert besonders gut miteinander verglichen werden.

Im Vergleich zu herkömmlichen Verfahren zur Geschlechtsbestimmung im Ei, insbesondere im Hühnerei, kann das Verfahren bereits nach einer zeitlich sehr kurzen Bebrütung des Eis, d.h. nach einer sehr geringen Anzahl an Bruttagen durchgeführt werden. Gleichzeitig kann eine sehr präzise Bestimmung des Geschlechts nichtinvasiv realisiert werden.

Weitere Vorteile, Merkmale und Einzelheiten der Erfindung ergeben sich aus der nachfolgenden Beschreibung eines bevorzugten Ausführungsbeispiels sowie anhand der Zeichnung. Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung wie definiert in den Ansprüchen 1-6 zu verlassen.

Die Zeichnung zeigt in:
- FIG 1: eine schematische und teilweise geschnittene Seitenansicht einer Vorrichtung gemäß einer ersten Ausführungsform zum Durchführen eines Verfahrens zum Bestimmen des Geschlechts wenigstens eines Embryos in einem Hühnerei, bei welchem mittels eines nichtinvasiven Ermittlungsverfahrens in Form von Magnetresonanzspektroskopie wenigstens ein die Konzentration von Östradiol in dem Hühnerei charakterisierender Östradiolwert ermittelt wird, wobei das Geschlecht des sich zukünftig gegebenenfalls entwickelnden Embryos in Abhängigkeit von dem ermittelten Östradiolwert bestimmt wird;
- FIG 2: eine schematische, perspektivische und geschnittene Seitenansicht der Vorrichtung gemäß einer zweiten Ausführungsform;
- FIG 3: eine schematische, perspektivische und geschnittene Seitenansicht der Vorrichtung gemäß einer dritten Ausführungsform;
- FIG 4: eine schematische Seitenansicht der Vorrichtung gemäß einer vierten Ausführungsform;
- FIG 5: die Strukturformel von Östradiol; und
- FIG 6: eine Tabelle mit der chemischen Verschiebung von Östradiol.

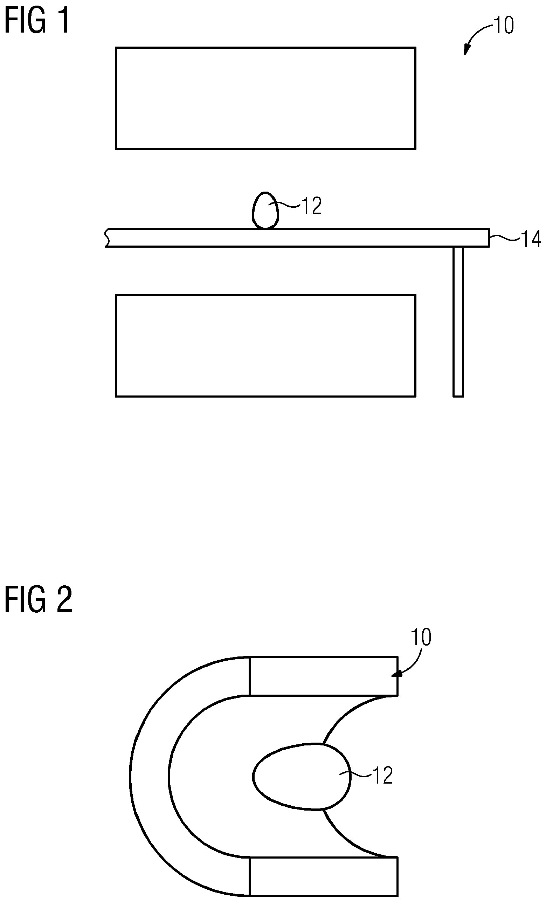

FIG 1 zeigt einen Kernspintomographen 10 gemäß einer ersten Ausführungsform, mittels welchem ein Ermittlungsverfahren in Form einer Magnetresonanzspektroskopie zur Bestimmung des Geschlechts eines Embryos in einem Ei 12 durchgeführt wird. Hierzu wird das Ei 12 auf einen Untersuchungstisch 14 im Kernspintomographen 10 angeordnet. Das Ermittlungsverfahren bzw. die Magnetresonanzspektroskopie ist dabei bezogen auf das Ei 12 nichtinvasiv. Dies bedeutet, dass zur Geschlechtsbestimmung die Schale des Eis 12 nicht geöffnet und keine Komponenten oder Substanzen aus dem Ei 12 entnommen werden. In FIG 1 ist lediglich das eine Ei 12 gezeigt. Selbstverständlich sind auch mehrere Eier gleichzeitig in dem Kernspintomographen 10 anordenbar und hinsichtlich des jeweiligen Geschlechts zu untersuchen.

Unter dem Bestimmen bzw. Untersuchen des Geschlechts des Embryos in dem Ei 12 ist zu verstehen, dass das Geschlecht des sich zukünftig gegebenenfalls entwickelnden Embryos in dem Ei 12 bestimmt wird. Mit anderen Worten muss der Embryo in dem Ei 12 nicht oder nicht vollständig entwickelt sein, um sein Geschlecht zu bestimmen. Dies bedeutet, dass als das Geschlecht das Geschlecht bestimmt wird, welches der Embryo hätte, wenn er sich entwickeln würde.

Das Verfahren ermöglicht es nämlich, das Geschlecht des sich zukünftig gegebenenfalls, also etwaig entwickelnden Embryos in einem besonders jungen Alter des Eis 12, d.h. besonders frühzeitig, zu bestimmen. Das Geschlecht wird beispielsweise ungefähr am siebzehnten Bruttag des Eis 12 bestimmt. Dies ist möglich, da - wie im Folgenden noch erläutert wird - nicht der noch gar nicht in dem Ei 12 vorhandene Embryo selbst mittels der Magnetresonanzspektroskopie untersucht wird. Mittels des nichtinvasiven Ermittlungsverfahrens in Form der Magnetresonanzspektroskopie wird wenigstens ein die Konzentration von Östradiol, insbesondere von 17β-Östradiol, in dem Ei 12 charakterisierender Östradiolwert ermittelt, wobei in Abhängigkeit von dem ermittelten Östradiolwert das Geschlecht bestimmt wird.

Wie aus FIG 2, 3 und 4 erkennbar ist, können unterschiedliche Ausführungsformen des Kernspintomographen 10 zur Geschlechtsbestimmung eingesetzt werden. Dabei wird das Ei 12 jeweils im sogenannten Sichtbereich des entsprechenden Kernspintomographen 10 angeordnet, um das Ermittlungsverfahren durchzuführen. Mit anderen Worten wird das Ei 12 in einem jeweiligen Erfassungsbereich des entsprechenden Kernspintomographen 10 angeordnet, um das Ei 12 mittels des Kernspintomographen 10 erfassen und den Östradiolwert ermitteln zu können.

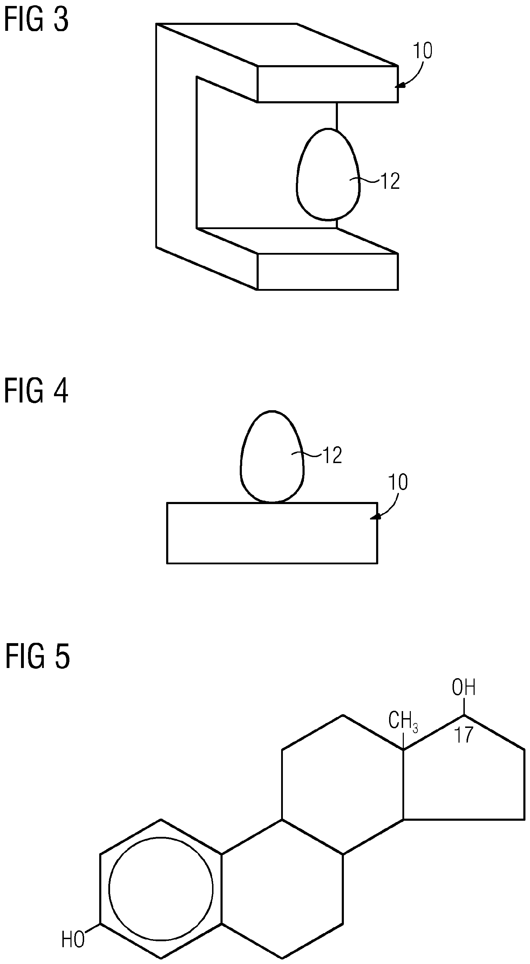

FIG 5 zeigt dabei die Strukturformel von 17β-Östradiol. Mittels der Magnetresonanzspektroskopie wird die chemische Verschiebung von Wasserstoff- und/oder Kohlenstoffatomen in dem Ei 12 in der Einheit Parts per Million (ppm) ermittelt. Hierzu wird beispielsweise eine ¹H- bzw. eine ¹³C-Spektroskopie durchgeführt.

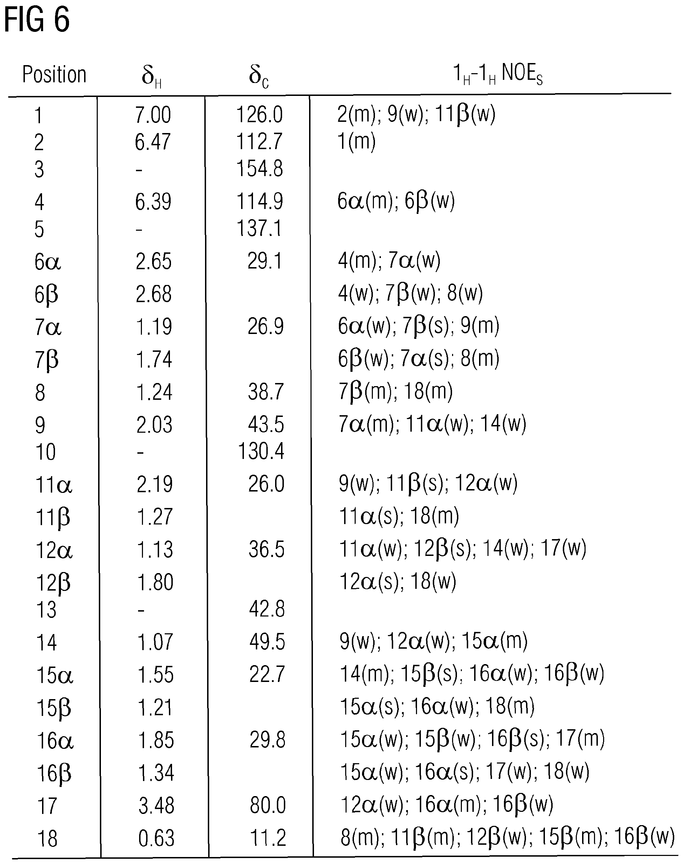

FIG 6 zeigt tabellarisch die chemische Verschiebung von 17β-Östradiol, wobei mit δ_{H} die chemische Verschiebung der H-Atome von 17β-Östradiol und mit δ_{C} die chemische Verschiebung der C-Atome von 17β-Östradiol bezeichnet ist. Mit NOEs sind die Kern-Overhauser-Effekte bezeichnet (NOE - Nuclear-Overhauser-Effect), wobei mit s starke Kern-Overhauser-Effekt-Intensitäten, mit m mittlere Kern-Overhauser-Effekt-Intensitäten und mit w schwache Kern-Overhauser-Effekt-Intensitäten bezeichnet sind. Darüber hinaus wurden die Kern-Overhauser-Effekt-Intensitäten umgewandelt in obere Grenzabstandsbedingungen von 2,7, 3,5 und 5,0 Ångström in der moleküldynamischen Berechnung.

Mittels der Magnetresonanzspektroskopie kann ferner zumindest bezogen auf das Ei 12 nichtinvasiv ein die Konzentration von Testosteron in dem Ei 12 charakterisierender Testosteronwert ermittelt werden. Schließlich wird ein Verhältnis des Östradiolwerts zum Testosteronwert gebildet, wobei in Abhängigkeit von diesem Verhältnis das Geschlecht bestimmt wird. Hierdurch kann das Geschlecht zu einem besonders frühen Zeitpunkt, d.h. nach einer nur sehr geringen Anzahl an Bruttagen des Eis 12 sowie mit einer besonders hohen Wahrscheinlichkeit korrekt bestimmt bzw. vorhergesagt werden. Die Ermittlung des weiblichen Geschlechtshormons Östradiol bzw. 17β-Östradiol und des männlichen Geschlechtshormons Testosteron ermöglichen dabei die Realisierung besonders aussagekräftiger Ergebnisse, so dass mit sehr hoher Wahrscheinlichkeit eine korrekte Aussage darüber getroffen werden kann, ob sich in dem Ei 12 ein männliches oder ein weibliches Küken entwickeln wird bzw. entwickeln würde.

Da das Verfahren vor der Entwicklung und vor dem Schlüpfen des Kükens durchgeführt werden kann, können in Abhängigkeit von dem ermittelten bzw. bestimmten Geschlecht auch besonders frühzeitig entsprechende Maßnahmen ergriffen werden, um die Entwicklung des Embryos bzw. des Kükens zu gewährleisten oder um die Entwicklung bzw. die weitere Entwicklung zu verhindern.

## Patentansprüche

1. Verfahren zum Bestimmen des Geschlechts wenigstens eines Embryos in einem Vogelei (12), bei welchem das Geschlecht des Embryos mittels wenigstens eines zumindest bezogen auf das Ei (12) nichtinvasiven Ermittlungsverfahrens bestimmt wird, wobei mittels des nichtinvasiven Ermittlungsverfahrens wenigstens ein die Konzentration von Östradiol in dem Ei (12) charakterisierender Östradiolwert ermittelt und in Abhängigkeit von dem Östradiolwert das Geschlecht bestimmt wird,
**dadurch gekennzeichnet, dass**
mittels des nichtinvasiven Ermittlungsverfahrens die chemische Verschiebung von Wasserstoffatomen und/oder von Kohlenstoffatomen im Ei (12) ermittelt und in Abhängigkeit von der ermittelten Verschiebung der Östradiolwert ermittelt wird.

2. Verfahren nach Anspruch 1,
**dadurch gekennzeichnet, dass**
als das nichtinvasive Ermittlungsverfahren ein bildgebendes Verfahren, insbesondere Magnetresonanzspektroskopie, durchgeführt wird.

3. Verfahren nach einem der vorhergehenden Ansprüche,
**dadurch gekennzeichnet, dass**
mittels des nichtinvasiven Ermittlungsverfahrens wenigstens ein die Konzentration von Testosteron in dem Ei (12) charakterisierender Testosteronwert und ein Verhältnis des Östradiolwerts zu dem Testosteronwert ermittelt wird, wobei das Geschlecht in Abhängigkeit von dem Vergleich bestimmt wird.

4. Verfahren nach einem der vorhergehenden Ansprüche,
**dadurch gekennzeichnet, dass**
der Östradiolwert vor dem dreißigsten Bruttag des Eis ermittelt wird.

5. Verfahren nach Anspruch 4,
**dadurch gekennzeichnet, dass**
der Östradiolwert vor dem fünfundzwanzigsten, insbesondere ungefähr am siebzehnten, Bruttag ermittelt wird.

6. Verfahren nach einem der Ansprüche 3 bis 5,
**dadurch gekennzeichnet, dass**
der Östradiolwert und der Testosteronwert am gleichen Bruttag des Eis ermittelt werden.

## Claims

1. Method for determining the sex of at least one embryo in a bird's egg (12), in which the sex of the embryo is determined by at least one determination method which is noninvasive at least in relation to the egg (12), where, by means of the noninvasive determination method at least one estradiol value which characterizes the concentration of estradiol in the egg (12) is determined and the sex being determined as a function of the estradiol value,
**characterized in that**
the shift of hydrogen atoms and/or carbon atoms in the egg (12) is determined by means of the noninvasive determination method and the estradiol value is determined as a function of the shift which has been determined.

2. Method according to Claim 1,
**characterized in that**
an imaging method, in particular magnetic resonance spectroscopy, is carried out as the noninvasive determination method.

3. Method according to any of the preceding claims,
**characterized in that**
at least one testosterone value which characterizes the concentration of testosterone in the egg (12) is determined by means of the noninvasive determination method and a ratio of the estradiol value to the testosterone value is determined, the sex being determined as a function of the comparison.

4. Method according to any of the preceding claims,
**characterized in that**
the estradiol value is determined before the thirtieth incubation day of the egg.

5. Method according to Claim 4,
**characterized in that**
the estradiol value is determined before the twentyfifth, in particular approximately on the seventeenth, incubation day.

6. Method according to any of Claims 3 to 5,
**characterized in that**
the estradiol value and the testosterone value are determined on the same incubation day of the egg.

## Revendications

1. Procédé pour déterminer le sexe d'au moins un embryon dans un oeuf d'oiseau (12), selon lequel on détermine le sexe de l'embryon au moyen d'au moins un procédé de détermination non invasif du moins par rapport à l'oeuf (12), au moins une valeur d'oestradiol caractérisant la concentration d'oestradiol dans l'oeuf (12) étant déterminée au moyen du procédé de détermination non invasif et le sexe étant déterminé en fonction de la valeur d'oestradiol,
**caractérisé en ce que**
au moyen du procédé de détermination non invasif on détermine le déplacement chimique d'atomes d'hydrogène et/ou d'atomes de carbone dans l'oeuf (12) et on détermine la valeur d'oestradiol en fonction du déplacement déterminé.

2. Procédé selon la revendication 1,
**caractérisé en ce que**
comme procédé de détermination non invasif on met en oeuvre un procédé d'imagerie, en particulier la spectroscopie par résonance magnétique.

3. Procédé selon l'une des revendications précédentes,
**caractérisé en ce que**
au moyen du procédé de détermination non invasif on détermine au moins une valeur de testostérone caractérisant la concentration de testostérone dans l'oeuf (12) et on détermine un rapport entre la valeur d'oestradiol et la valeur de testostérone, le sexe étant déterminé en fonction de la comparaison.

4. Procédé selon l'une des revendications précédentes,
**caractérisé en ce que**
on détermine la valeur d'oestradiol avant le trentième jour d'incubation de l'oeuf.

5. Procédé selon la revendication 4,
**caractérisé en ce que**
on détermine la valeur d'oestradiol avant le vingt-cinquième jour, plus particulièrement environ au dix-septième jour d'incubation.

6. Procédé selon l'une des revendications 3 à 5,
**caractérisé en ce que**
on détermine la valeur d'oestradiol et la valeur de testostérone le même jour d'incubation de l'oeuf.
